Europäisches Patentamt

European Patent Office

Office européen des brevets

(11) Veröffentlichungsnummer: **0 217 041**
**A1**

## (12) EUROPÄISCHE PATENTANMELDUNG

(21) Anmeldenummer: 86110271.3

(51) Int. Cl.⁴: **A61K 7/16 , A61K 7/26**

(22) Anmeldetag: 25.07.86

(30) Priorität: 09.08.85 DE 3528709

(43) Veröffentlichungstag der Anmeldung:
08.04.87 Patentblatt 87/15

(84) Benannte Vertragsstaaten:
AT BE CH DE FR GB IT LI LU NL SE

(71) Anmelder: Schleicher, Peter, Dr. med.
Ismaninger Strasse 65
D-6500 München 80(DE)

(72) Erfinder: Schleicher, Peter, Dr. med.
Ismaninger Strasse 65
D-6500 München 80(DE)

(74) Vertreter: Weickmann, Heinrich, Dipl.-Ing. et
al
Patentanwälte Dipl.-Ing. H.Weickmann
Dipl.-Phys.Dr. K.Fincke Dipl.-Ing.
F.A.Weickmann Dipl.-Chem. B. Huber Dr.-Ing.
H. Liska Dipl.-Phys.Dr. J. Prechtel Postfach
860820
D-8000 München 86(DE)

(54) Mundpflegemittel.

(57) Die Erfindung betrifft Mundpflegemittel, insbesondere Zahnpasten, Mundwässer und medizinische Kaugummis, die wohlschmeckende Benzopyrone, im besonderen Cumarine, zur Verbesserung der Mundhygiene sowie zur Steigerung der Aktivität von natürlichen Killerzellen des Immunsystems enthalten.

EP 0 217 041 A1

## Mundpflegemittel

Die Erfindung betrifft Mundpflegemittel, insbesondere Zahnpasten, Mundwässer und medizinische Kaugummis.

Mundpflegemittel haben nicht nur allein eine kosmetische Aufgabe, sondern dienen auch der Hygiene im Sinne der Erhaltung natürlicher biologischer Verhältnisse in der Mundhöhle. Zur umfangreichen Gruppe der Mundpflegemittel gehören Pasten, Gele, Pulver and medizinische Kaugummis. Die Bedeutung der täglichen Mundpflege wird allgemein anerkannt, aber nicht von jedermann mit der notwendigen Konsequenz durchgeführt. Es ist daher ein Ziel der Erfindung, die regelmäßige Anwendung von Mundpflegemitteln zu erhöhen.

Ein weiteres Ziel der Erfindung ist es, durch ein einfaches täglich angewendetes Verfahren das Immunsystem biologisch zu stimulieren und zu modulieren.

Diese Ziele werden erfindungsgemäß gelöst durch ein Mundpflegemittel, welches ein Benzopyron enthält. Da Cumarine in vielen Pflanzen enthalten sind, können sie auch anstatt als Reinsubstanz dem Mundpflegemittel in Form von Pflanzenteilen bzw. deren Extrakten, zugesetzt werden. Erfindungsgemäß könnten solche Benzopyrone enthalten sein, die zumindest einen angenehmen Geschmack aufweisen. Ein bevorzugter Vertreter dieser Substanzgruppe, das 5,6-Benzo-alpha-pyron (Cumarin) ist z. B. im Steinklee (Melilotus) oder im Waldmeister (Galium odoratum) enthalten und kann daraus durch Extraktion gewonnen werden (siehe DAB). Cumarin ist aber auch leicht aus Salicylaldehyd' und Acetanhydrid durch eine Perkinreaktion und einer spontanen Wasserabspaltung herstellbar (Bayer, Lehrbuch der organischen Chemie, Seite 472). Cumarinderivate bzw. Benzopyrone sind in der Literatur häufig beschrieben (z. B. Campbell et al., J. Biol. Chem. 138, 21, 1941 oder der Cumarin 3-Carbonsäureethylester, Organikum, Org. Chem. Grundprakt. Seite 509).

Der angenehme Geruch und der Geschmack nach Waldmeister, den Cumarine aufweisen, fördert die häufige Anwendung von Mundpflegemitteln; die solche Substanzen enthalten. Dadurch läßt sich ein Ziel der Erfindung, die Steigerung der Mundhygiene durch häufigere Pflege, erreichen.

Cumarine zeigen auch eine ausgeprägte Steigerung der Aktivität von natürlichen Killerzellen des Immunsystems. (Wattenberg et al, Cancer Res. 39, 1651, (1976)). Ausgenommen davon sind allerdings die Dicumarine, die in der Literatur auch als Vitamin K Antagonisten beschrieben sind und in der Schulmedizin häufig zu den Cumarinen gezählt werden. Der Mechanismus, über den die antitumoröse Wirkung erreicht wird, ist unbekannt. An einkernigen Zellen, die von Tumorpatienten gewonnen wurden, konnte gezeigt werden, daß eine 18stündige Inkubation dieser Zellen mit 5,6-Benzo-alpha-Pyron eine 20 bis 25%ige Steigerung der natürlichen Cytotoxizität gegenüber Melanomzellen und Gliomzellen bewirkt (Zänker K.S. et al, Drugs Exptl. Clin. Res. X(11) 767-774 (1984)). Außerdem konnte gezeigt werden, daß die Zeit zwischen der chirurgischen Entfernung eines Primär-Tumors und dem Entstehen von Secundär-Geschwulsten durch eine Gabe von 100 mg 5,6-Benzo-alpha-Pyron pd verlängert wird. Es·hat sich erwiesen, daß Tagesdosen von mindestens 5 mg und bis zu 400 mg gut wirksam und verträglich sind. Bevorzugt werden Dosen von 20 bis 250 mg pro Tag verabreicht, wobei die am meisten bevorzugte Dosis 45 mg pro Tag beträgt.

Durch Beimischung dieser Substanz wird ein weiteres Ziel der Erfindung, die Stimulierung des Immunsystems, erreicht.

Die folgenden Beispiele sollen die Erfindung näher erläutern:

Beispiel 1

4,3 kg Calciumcarbonat und 200 g Silikagel werden in einer Trommel zu einem Putzkörperpulver gemischt und anschließend durch ein feines Sieb gedrückt. Gleichzeitig wird ein Zahnpastaschleim aus 3,5 kg Wasser und 1,5 kg Glycerin sowie 80 g Natriumalginat in einem Rührwerk vermischt. Danach werden die feingesiebten, wasserunslöslichen Putzkörper in den Zahnpastenschleim eingetragen und diese Mischung so lange gerührt, bis sie gleichmäßig fest ist und eine pastenähnliche Konsistenz erreicht hat. Anschließend werden 200 g Na-Laurylsulfat als Schaummittel und 500 g 5,6-Benzopyron als Geschmacks-und als Wirkstoff zugegeben, wobei die Rührgeschwindigkeit vermindert wird, um die einsetzende Schaumentwicklung zu vermindern.

Beispiel 2

Analog dem Beispiel 1 wird eine Zahnpasta aus folgenden Substanzen hergestellt:

4 kg $CaHPO_4 \cdot 2H_2O$ und 0,6 kg $CaHPO_4$ als Putzkörper, 100 g Algitex als Bindemittel, 100 g

TiO$_2$ als Weißmacher, 2,8 kg Glycerin, 2,0 kg Wasser, 30 g Paraffinöl, 10 g PHD Ethylester und 0,6 kg 5,6-Benzo-alpha-Pyron.

Beispiel 3

Ein Mundwasser, bestehend aus folgenden Substanzen:

400 g H$_2$O,

450 g Tween 80,

50 g 5,6-Benzo-alpha-Pyron.

Anwendung: 3 Tropfen auf ½ Glas Wasser, damit zwei bis dreimal täglich die Mundhöhle kräftig ausspülen.

**Ansprüche**

1. Mundpflegemittel, **gekennzeichnet durch** einen Gehalt an Cumarin bzw. Cumarinderivaten.

2. Mundpflegemittel nach Anspruch 1, **dadurch gekennzeichnet,** daß es das Cumarinderivat in Form von Cumarinderivat enthaltenden Pflanzenteilen enthält.

3. Mundpflegemittel nach einem der vorhergehenden Ansprüche, **dadurch gekennzeichnet,** daß es Extrakte aus Cumarinderivat enthaltenden Pflanzenteilen enthält.

4. Mundpflegemittel nach einem der vorhergehenden Ansprüche, **dadurch gekennzeichnet,** daß das Cumarinderivat das 5,6-Benzo-alpha-Pyron ist.

5. Mundpflegemittel nach einem der vorhergehenden Ansprüche, **dadurch gekennzeichnet,** daß das Cumarinderivat in einer Menge enthalten ist, die bei regelmäßiger Mundpflege einer Tagesdosis von 5 bis 400 mg entspricht.

6. Mundpflegemittel nach Anspruch 5, **dadurch gekennzeichnet,** daß das Cumarinderivat in einer Menge enthalten ist, die bei regelmäßiger Anwendung des Pflegemittels einer Tagesdosis von 45 mg entspricht.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| X | DE-A-1 492 185 (C. STRALFORS) * Ansprüche; Seite 4, Zeilen 3-10; Seite 5, Tabelle * | 1,4-6 | A 61 K 7/16 A 61 K 7/26 |
| X | FR-M- 1 231 (L. ROMANO) * Seite 1, linke Spalte, Zeile 1 - rechte Spalte, Zeile 10 * | 1,2 | |
| X | CHEMICAL ABSTRACTS, Band 82, 1975, Seite 277, Zusammenfassung Nr. 175146x, Columbus, Ohio, US; & JP-A-75 13 546 (SUNSTAR DENTIFRICE CO. LTD.) 13-02-1975 * Zusammenfassung * | 1,3 | |
| X | DE-C- 305 722 (G. EICHELBAUM) * Anspruch; Beispiel * | 1,4,5 | **RECHERCHIERTE SACHGEBIETE (Int. Cl.4)** |
| X | US-A-3 223 588 (F. PICKEL) * Ansprüche * | 1 | A 61 K |
| X | US-A-3 887 701 (J. NACHTIGAL) * Anspruch 1; Beispiel 1 * | 1,5 | |

Der vorliegende Recherchenbericht wurde für alle Patentansprüche erstellt.

| Recherchenort | Abschlußdatum der Recherche | Prüfer |
|---|---|---|
| DEN HAAG | 12-01-1987 | WILLEKENS G.E.J. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82